# EUROPÄISCHE PATENTANMELDUNG

(11) **EP 1 178 027 A1**
(43) Veröffentlichungstag der Anmeldung: **06.02.2002**
(21) Anmeldenummer: 01116167.6
(22) Anmeldetag: 04.07.2001
(51) Int. Cl.: C07C 7/00

(54) **Verfahren und Vorrichtung zur Entfernung von sauren und/oder basischen Bestandteilen aus Kohlenwasserstoffen**

(30) Priorität: 05.08.2000 DE 10038320
(71) Anmelder: Krupp Uhde GmbH, 44141 Dortmund (DE)
(72) Erfinder: Emmrich, Gerhard, 45133 Essen (DE); Ennenbach, Frank, 76344 Eggenstein (DE); Ranke, Uwe, Dr., 45128 Essen (DE); Gehrke, Helmut, Dr., 59269 Beckum-Vellern (DE)

(57) **Zusammenfassung**

Die Erfindung betrifft ein Verfahren zur Reinigung eines Kohlenwasserstoffdampfes, der wenigstens aus einer aromatischen, einer olefinischen, einer paraffinischen Verbindung oder aus einer daraus gebildeten Mischung besteht und welcher Kohlenwasserstoffdampf verunreinigende Bestandteile mit sich führt, die sauren und/oder basischen Charakter besitzen und welche Bestandteile wenigstens aus einem wasserlöslichen, organischen und/oder anorganischen Stoff bestehen und eine Vorrichtung zur Durchführung des Verfahrens.

Im verfahrensgemäß erzeugten Produkt kann bezüglich Verunreinigungen organischer und anorganischer Verbindungen der Elemente Schwefel, Stickstoff, Sauerstoff und Chlor eine extrem hohe Reinheit erreicht werden. Hierzu wird in den Kohlenwasserstoffdampf in einer Mischzone (5) eine wässrige Lösung (7) dispers eingebracht, der dadurch gebildete zweiphasige, mit Tröpfchennebel beladene Dampf (8) wird dann unmittelbar in eine Kondensationszone (6) übergeleitet, worin er mit beiden Phasen gemeinsam niederschlägt. Die dabei entstandene Kondensat-Emulsion (9) wird in eine Abscheidezone (10) eingeleitet, in der die im Wesentlichen gereinigten Kohlenwasserstoff enthaltende eine Teilflüssigphase (12) der Emulsion von der im Wesentlichen Lösungswasser mit den darin gelösten verunreinigenden Bestandteilen enthaltenden anderen Teilflüssigphase (15) der Emulsion abgetrennt wird

Der Zusammenfassung ist die Figur beizufügen.

## Beschreibung

Die Erfindung betrifft ein Verfahren und eine Vorrichtung zur Reinigung eines Kohlenwasserstoffdampfes, der wenigstens aus einer aromatischen oder einer olefinischen oder einer paraffinischen Verbindung oder aus einer daraus gebildeten Mischung besteht und welcher Kohlenwasserstoffdampf verunreinigende Bestandteile mit sich führt, die sauren und/oder basischen Charakter besitzen und welche Bestandteile wenigstens aus einem wasserlöslichen, organischen und/oder anorganischen Stoff bestehen. Im verfahrensgemäß erzeugten Produkt kann bezüglich Verunreinigungen organischer und anorganischer Verbindungen der Elemente Schwefel, Stickstoff, Sauerstoff und Chlor eine extrem hohe Reinheit erreicht werden.

Die Freiheit von Verunreinigungen in Kohlenwasserstofffraktionen ist ein seit langem bestehendes Bedürfnis der verarbeitenden Industrie. Hierbei wird gewünscht, auch kleinste Verunreinigungen organischer und anorganischer Verbindungen der Elemente Schwefel, Stickstoff, Sauerstoff und Chlor bis hin in den ppb-Bereich zu entfernen, damit sensitiver und selektiver wirkende Katalysatorsysteme und hierbei besonders Katalysatoren auf Zeolithbasis zum Einsatz kommen können. So wird z. B. für die neuen Katalysatoren zur Ethylbenzolsynthese im Benzol ein maximaler Gehalt an organisch gebundenem Stickstoff von 30 ppb gefordert, wobei die Angabe ppb auf die Masse bezogen ist, wie auch bei allen folgenden ppm- und ppb-Angaben in dieser Schrift.

Verunreinigungen der eingangs erwähnten Art finden sich als Spurenverunreinigungen im ppm-Bereich mit Schwefel-, Sauerstoff-, Stickstoff-, Chlorverbindungen sowohl in Produktfraktionen nach Destillationen, Absorptionen, Adsorptionen, Extraktionen und Extraktivdestillationen. Sie resultieren zum einen aus Resten von Extraktions- oder Lösungsmitteln oder deren Zersetzung, zum anderen aus Stoffen, die mit dem Einsatzprodukt in die Anlage gelangt sind oder sich durch Reaktion in der Anlage gebildet haben.

Im bisherigen Stand der Technik werden solche unerwünschten Bestandteile, wenn sie basisch reagieren, mit sauer aktivierter Bleicherde aus dem danach fertigen Produkt entfernt. Eine solche Bleicherde-Behandlung bringt jedoch folgende, bekannte Nachteile mit sich:
- die Bleicherde hat nur eine begrenzte Beladungskapazität
- der Zeitpunkt des Durchbruches kann nicht exakt vorhergesagt werden
- es sollten mindestens stets zwei Bleicherdetürme parallel vorhanden sein
- die Bleicherde kann nicht regeneriert werden
- nach der Beladung muss die Bleicherde kohlenwasserstofffrei gedämpft werden
- die gedämpfte Bleicherde muss bergmännisch aus dem Turm abgebaut werden
- die Bleicherde muss geglüht werden, um etwaige verbliebene Kohlenwasserstoffe zu entfernen
- die geglühte Bleicherde muss deponiert werden.

Sauer reagierende, verunreinigende Bestandteile werden nach dem jetzigen Stand der Technik mit aktivierter Kohle, kaustischer Soda oder lonenaustauscherharzen aus den Kohlenwasserstoffen entfernt.

Bezüglich der Zugabe von Wasser in dampfförmige Kohlenwasserstoffe gibt die US 4,168,209 an, Wasser oberhalb der Extraktionsmittelzugabe in eine Destillationskolonne für die Extraktivdestillation einzugeben, das Kopfprodukt dieser Kolonne zu kondensieren und die sich ergebenden Phasen zu trennen. Im Unterschied zu der vorliegenden Erfindung dient diese Wasserzugabe jedoch nicht zur Reinigung der Kohlenwasserstoffe von unerwünschten Bestandteilen, sondern zur Minimierung der Extraktionsmittelverluste, hauptsächlich bereits innerhalb der Destillationskolonne, in die das Wasser eingeführt wird. Diese US 4,168,209 gibt auch nicht an, welche Reinheit erreicht werden könnte. Ein weiterer Unterschied der US 4,168,209 zu der vorliegenden Erfindung ist der Ort der Wasserzugabe. Die vorliegende Erfindung sieht die Zugabestelle unmittelbar vor dem Kondensator vor und nicht in eine vorgeschaltete Kolonne, daher ist die vorliegende Erfindung unabhängig von Destillationskolonnen einsetzbar und es kann bei dem erfindungsgemäßen Reinigungsverfahren auch kein Wasser bzw. keine wässrige Lösung in eine Kolonne zurückfließen.

Das erfindungsgemäße Verfahren besitzt die Aufgabe, die Nachteile dieser Behandlungsverfahren und der bekannten Vorrichtungen zu vermeiden und ein wirtschaftliches Verfahren zur Reinigung von Kohlenwasserstoffgemischen zur Verfügung zu stellen, das auch kleinste Verunreinigungen organischer Verbindungen der Elemente Schwefel, Stickstoff, Sauerstoff und Chlor bis in den ppb-Bereich hinein entfernen kann. Das erfindungsgemäße Verfahren dient zur Reinigung eines Kohlenwasserstoffdampfes, der wenigstens aus einer aromatischen oder einer olefinischen oder einer paraffinischen Verbindung oder aus einer daraus gebildeten Mischung besteht und welcher Kohlenwasserstoffdampf verunreinigende Bestandteile mit sich führt, die sauren und/oder basischen Charakter besitzen und welche Bestandteile wenigstens aus einem wasserlöslichen, organischen und/oder anorganischen Stoff bestehen.

Die Lösung dieser Aufgaben ist im Kennzeichen des Hauptanspruches wie auch des Vorrichtungsanspruches 10 enthalten. Die Unteransprüche 2 bis 10 enthalten sinnvolle Ergänzungen dazu.

Im erfindungsgemäßen Verfahren wird zu reinigender Kohlenwasserstoffeinsatz zunächst verdampft, sofern er nicht bereits dampfförmig vorliegt, etwa als Kopfprodukt einer Destillations- bzw. Abtriebskolonne. Eine Überhitzung soll dabei vermieden werden oder zumindest gering gehalten werden, damit der gebildete Kohlenwasserstoffdampf überwiegend als gesättigter Dampf vorliegt. In diesen Kohlenwasserstoffdampf wird in einer Mischzone direkt eine wässrige Lösung dispers eingebracht, zum Beispiel eingedüst. Die Lösungswassermenge, bezogen auf den Kohlenwasserstoffdampf, kann zwischen 1 Gew.% und 20 Gew.% betragen, bevorzugt sind 5 Gew.%. Zumindest ein Teil der wässrigen Lösung wird dabei verdampft. Dadurch wird dem Kohlenwasserstoffdampf Wärme entzogen, was bewirkt, dass ein Teil der Kohlenwasserstoffe aus der Dampfphase kondensiert und sich mit den Tröpfchen der eingedüsten wässrigen Lösung intensiv mischt, wobei ein erster Teil der unerwünschten Bestandteile aus der Kohlenwasserstoffphase in die wässrige Phase, in denen sie sich leichter lösen, entsprechend dem Verhältnis ihrer Löslichkeiten überwechseln.

Dadurch entsteht ein zweiphasiger, nämlich mit Tröpfchennebel beladener Dampf. Seine Gasphase enthält die Dämpfe der eingesetzten Kohlenwasserstoffe sowie Wasserdampf. Seine Flüssigphase enthält den eingedüsten Tröpfchennebel des Lösungswassers mit den darin gelösten verunreinigenden Bestandteilen. Die Zusammensetzung der Flüssigphase und deren Wasseranteil ist dabei abhängig von den Siedepunkten der eingesetzten Kohlenwasserstoffe. Besteht der zu reinigende Kohlenwasserstoffeinsatz z. B. im Wesentlichen aus Benzol, um aus ihm Reinbenzol zu erzeugen, so wird der Wasseranteil im Tröpfchennebel überwiegen, weil der Siedepunkt des Wassers mit 100 °C oberhalb des Siedepunktes von Benzol (80,1 °C) liegt. Wird jedoch die wässrige Lösung in einen zu reinigenden Kohlenwasserstoffeinsatz eingedüst, der z. B. im Wesentlichen aus Toluol mit einem Siedepunkt von 110 °C besteht oder aber der z. B. im Wesentlichen aus einem Gemisch des Ethylbenzols mit Xylolen besteht, welches Gemisch einen Siedebereich von 131 °C bis zu 144 °C aufweist, so wird der zweiphasige Dampf eine Temperatur oberhalb des Siedepunktes des Wassers annehmen und somit wird in diesem Falle der Kohlenwasserstoffanteil im Tröpfchennebel überwiegen. Der mit Tröpfchennebel beladene Dampf wird aus der Mischzone unmittelbar in eine Kondensationszone übergeleitet. In der Kondensationszone werden beide Phasen mit Kühlflächen in Kontakt gebracht, woran sie gemeinsam niedergeschlagen und in eine Kondensat-Flüssigkeit gewandelt werden, die als Emulsion der einen in der anderen Flüssigkeit anfällt.

In der Kondensationszone findet abermals eine intensive Vermischung der beiden Phasen statt und bewirkt, dass der andere Teil der verunreinigenden Bestandteile der Kohlenwasserstoffphase in die wässrige Phase, in der sie sich leichter lösen, entsprechend dem Verhältnis ihrer Löslichkeiten überwechseln kann. Die in der Kondensationszone gebildete Kondensatflüssigkeit besteht aus der einen, im Wesentlichen Kohlenwasserstoff enthaltenden Teilflüssigphase und einer anderen, im Wesentlichen das Lösungswasser enthaltenden Teilflüssigkeitsphase. Die Kondensatflüssigkeit wird aus der Kondensationszone abgezogen und in eine Abscheidezone eingeleitet.

In der Abscheidezone wird die eine, im Wesentlichen Kohlenwasserstoff enthaltende Teilflüssigphase von der anderen, im Wesentlichen das Lösungswasser mit den darin gelösten verunreinigenden Bestandteilen enthaltenden Teilflüssigphase abgetrennt. Die Abtrennung der einen Teilflüssigkeit von der anderen Teilflüssigkeit geschieht mittels Ausnutzung des unterschiedlichen spezifischen Gewichts beider Teilflüssigphasen, z. B. durch Schwerkraft oder Zentrifugalkraft oder andere vergleichbare Mittel. Daher ist die vorliegende Erfindung dahingehend ausgestaltet, dass die Abtrennung der einen, im Wesentlichen gereinigten Kohlenwasserstoff enthaltenden Teilflüssigphase der Emulsion von der anderen, im Wesentlichen Lösungswasser enthaltenden Teilflüssigphase der Emulsion mittels eines Wasserabscheiders erfolgt.

Die Kohlenwasserstoffphase ist gereinigt und muss, falls ihre Wasserfreiheit für die spätere Verwendung gefordert wird, noch getrocknet werden. Die wässrige Phase wird üblicherweise, aber nicht notwendigerweise, in zwei Teilströme aufgespalten: Der eine Teilstrom wird biologisch gereinigt und entsorgt. Der andere Teilstrom wird zur Eindüsungsstelle zurückgeführt und bildet somit einen Kreislauf. Das Verhältnis der beiden Teilströme ergibt sich aus dem Gehalt der gelösten Schadstoffe und der Ansprüche an die Reinheit des Kohlenwasserstoff-Produkts im einzelnen Anwendungsfall. Der ausführende Fachmann wird hierzu Laborversuche anstellen. Hierbei kann sich im Grenzfall auch ergeben, dass nur reines Frischwasser eingedüst werden darf, und dass die im Wasserabscheider abgetrennte wässrige Lösung vollständig entsorgt werden muss. Daher geschieht eine weitere Ausgestaltung der vorliegenden Erfindung, bei der zumindest ein Teil des aus der Emulsion abgetrennten Lösungswassers in einem Kreislauf rückgeführt wird und in die besagte Mischzone als Bestandteil der wässrigen Lösung dispers eingebracht wird und bei der mit dem verbliebenen Rest des aus der Emulsion abgetrennten Lösungswassers die besagten verunreinigenden Bestandteile aus dem Lösungswasserkreislauf ausgeschleust und ihrer Entsorgung zugeführt werden.

Der vorteilhafte Einsatz des erfindungsgemäßen Verfahrens soll am Beispiel einer Extraktivdestillation zur Erzeugung hochreinen Benzols mittels des stickstoffhaltigen Extraktionsmittels N-Formylmorpholin näher erläutert werden, wobei der Einsatz selbstverständlich nicht auf Extraktivdestillationen oder auf die Entfernung von N-Formylmorpholin oder auf die Reinigung von Benzol beschränkt ist.

Das beispielgebende Extraktivdestillationsverfahren umfasst in der Regel mindestens zwei Kolonnen, eine Extraktivdestillationskolonne und eine nachgeschaltete Abtriebskolonne, beide können auch als Trennwandkolonne bzw. gradierende Kolonne baulich zusammengefasst sein. In der ersten Kolonne, der Extraktivdestillationskolonne, wird aus dem Einsatzprodukt, in diesem Falle einer Benzolfraktion, mittels eines selektiven Lösungsmittels, hier N-Formylmorpholin, das Benzol ausgewaschen. Die Nichtaromaten werden über Kopf gestrippt, während das Benzol zusammen mit dem Lösungsmittel zum Sumpf der Kolonne fließt. In der zweiten Kolonne; der Abtriebskolonne, werden Benzol und Lösungsmittel voneinander getrennt. Das abgetrennte Lösungsmittel sammelt sich im Sumpf der Abtriebskolonne und wird zum Wiedereinsatz zurück zum Kopf der Extraktivdestillationskolonne gepumpt. Das Benzol tritt dampfförmig am Kopf der Abtriebskolonne aus. Beim bisherigen Stand der Technik wird es dann ohne weiteres kondensiert und gesammelt im Rückflussbehälter, von wo ein Teil als Rückfluss zur Abtriebskolonne gepumpt wird. Der Rest des Benzols wird aus dem Rückflussbehälter als Fertigprodukt zur Anlagengrenze gefördert. Der Lösungsmittelrestgehalt im herkömmlichen Stand der Technik beträgt im Durchschnitt 1 ppm (entspricht 1000 ppb) N-Formyl-Morpholin (NFM) oder das Hydrolyseprodukt Morpholin, wobei diese beiden Stoffe zu etwa 1/7, bezogen auf ihre Masse, aus Stickstoff bestehen.

Das erfindungsgemäße Verfahren ermöglicht es, den Stickstoffgehalt im Fertigprodukt durch Eindüsen von Lösungswasser, gegebenenfalls zusammen mit Ameisensäure, welche gemeinsam in den Dampfstrom des Benzols vom Kopf der Abtriebskolonne unmittelbar vor dem Kondensator eingedüst werden, überraschenderweise auf einen Gehalt von kleiner 30 ppb zu verringern. Dies liegt daran, dass der Verteilungsfaktor im Dreistoffsystem NFM/Morpholin-Benzol-Wasser 30-mal größer für NFM/Morpholin-Wasser als für NFM/Morpholin-Benzol ist. Die Löslichkeit von Wasser im Benzol und von Benzol im Wasser ist sehr gering (bei 50 °C 1,3 g Benzol/1000 g Wasser und 1,56 g Wasser/1000 g Benzol). Daher findet im Anschluss an die intensive Vermischung eine Phasentrennung statt und das Lösungsmittel NFM befindet sich in der wässrigen Phase. Das erfindungsgemäße Verfahren besitzt daher den überzeugenden Vorteil, mit einfachen Mitteln die Herstellung eines hochreinen Produktes verfügbar zu machen.

Mit einer anderen Ausgestaltung der vorliegenden Erfindung kann die Produktreinheit über das vorerwähnte Maß hinaus erhöht werden, indem zumindest ein Teil der in die Mischzone dispers eingebrachten wässrigen Lösung aus Frischwasser besteht.

In weiterer Ausgestaltung der vorliegenden Erfindung wird die im Kreis geführte wässrige Lösung durch Zusatz einer Säure, beispielsweise Ameisensäure, auf einen pH-Wert geringfügig oberhalb 7, etwa bei 7,5 eingestellt, um die bereits im Kreislaufwasser vorhandenen Stickstoffverbindungen als Salz aus dem Lösungsgleichgewicht zu entfernen. Hierzu wird der in die Mischzone dispers eingebrachten wässrigen Lösung eine Säure beigemischt. Das erfindungsgemäße Verfahren wird hierzu im Einzelnen so ausgestaltet, dass als zur wässrigen Lösung beigemischte Säure die Ameisensäure eingesetzt wird.

In einer anderen Ausgestaltung der vorliegenden Erfindung ist vorgesehen, dass die nach der Säurezugabe in der wässrigen Lösung ausgefällten Salze durch Abscheidemittel aus der wässrigen Lösung entfernt werden.

Weiterhin besitzt das erfindungsgemäße Verfahren eine Ausgestaltung, nach der die Säurezugabe pH-Wert-geregelt erfolgt.

Bei der vorliegenden Erfindung sieht eine spezielle Verfahrensausgestaltung vor, die wässrige Lösung vor ihrem dispersen Einbringen in die Mischzone zu kühlen.

Auch besitzt die vorliegende Erfindung noch eine Verfahrensausgestaltung, bei der die in der Kondensationszone entstandene Kondensat-Emulsion vor ihrer Einleitung in die Abscheidezone unterkühlt wird.

Mit der vorliegenden Erfindung wird zugleich eine Vorrichtung zur Durchführung des erfindungsgemäßen Verfahrens bereitgestellt. Wie voranstehend beschrieben, sieht das erfindungsgemäße Verfahren vor, die wässrige Lösung unmittelbar vor dem Kondensator in den Kohlenstoffdampf dispers einzubringen, z. B es einzudüsen. Als besonders wirkungsvoll in Bezug auf die erzielbaren Produktreinheiten hat es sich gezeigt, die Mischzone und die Kondensationszone unter Vermeidung irgend einer zwischengeschalteten Überleitungsstrecke direkt miteinander in einer integralen Apparatekonstruktion zu verbinden. So weist eine Ausgestaltung der erfindungsgemäßen Vorrichtung einen Einzelapparat auf, in welchem die Mischzone und die Kondensationszone in einem beide Zonen einhüllenden gemeinsamen Raum angeordnet sind und welcher Raum von dem Mantel des Einzelapparates integrierend umgrenzt wird.

Das Verfahren eignet sich darüber hinaus auch zur nachträglichen Installation in bestehende Anlagen, da überwiegend die Rückflussbehälter in Fraktionierungen, Extraktionen und Extraktivdestillationen mit Wasserabscheidevorrichtungen ausgestattet sind oder sich mit geringem Aufwand damit ausstatten lassen.

Das anfallende, von den Verunreinigungen befreite Reinprodukt fällt wassergesättigt an (Wasser im Benzol bei 50 °C 1,56 g/1000 g). Muss das Reinprodukt wegen nachfolgender Synthesen wasserfrei sein, so lässt sich leicht eine destillative oder adsorptive Trocknung anschließen.

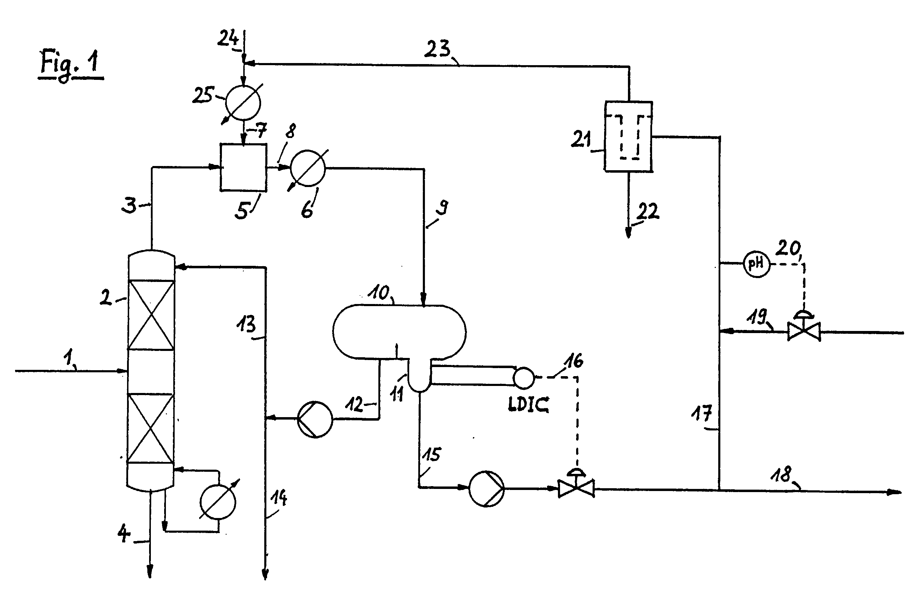

Das erfindungsgemäße Verfahren wird in Figur 1 beispielsweise näher beschrieben.

Dargestellt ist eine Anlage zur Reinigung von Benzol, das zuvor mittels Extraktivdestillation gewonnen wurde, wovon noch die Abtriebskolonne 2 gezeigt ist. Als Lösungsmittel wurde N-Formyl-Morpholin verwendet, die Reste dieses Lösungsmittels im Benzol stellen im Wesentlichen die verunreinigenden Bestandteile dar, die abgeschieden werden sollen.

Das von der Extraktivdestillationskolonne kommende Benzol-LösungsmittelGemisch wird über die Leitung 1 in die Abtriebskolonne 2 gegeben. In der Abtriebskolonne 2 erfolgt die Abtrennung des Benzols vom Lösungsmittel, wobei das Benzol als dampfförmiges Kopfprodukt über die Leitung 3 aus der Abtriebskolonne 2 abgezogen wird. Das von Benzol befreite Lösungsmittel wird aus dem Sumpf der Abtriebskolonne 2 entfernt und über die Leitung 4 zwecks Wiederverwendung zur Extraktivdestillationskolonne zurückgeführt.

Unmittelbar vor Eintritt der Benzoldämpfe in den Kondensator 6 wird in der Mischzone 5, die als Sprühvorrichtung ausgeführt ist, eine wässrige Lösung 7 eingedüst. Als wässrige Lösung 7 ist eine Mischung aus vollentsalztem Wasser, Wasserdampfkondensat, zurückgeführter wässriger Lösung und Ameisensäure vorgesehen. Diese wässrige Lösung verdampft zum Teil in der Mischzone 5. Die hierbei dem Benzoldampf entzogene Energie bewirkt eine Teilkondensation von Benzol in der Leitung 8. Im anschließenden Kondensator 6 wird die verdampfte wässrige Lösung zusammen mit dem restlichen Benzoldampf kondensiert und fällt zusammen mit den bereits kondensierten Tröpfchen im Kondensator 6 aus. Sowohl bei der Teilkondensation nach der Eindüsungsstelle in der Mischzone 5 für die wässrige Lösung als auch im Kondensator 6 findet eine intensive Mischung von Benzol und wässriger Lösung statt. Dabei wäscht die wässrige Lösung aus dem Kondensierten den größten Teil der Verunreinigungen aus.

Das kondensierte Benzol fließt mit der wässrigen Lösung über die Leitung 9 in den Rückflussbehälter 10, der mit einer Wasserabscheidevorrichtung 11 ausgestattet ist. Das gereinigte Benzol wird über die Leitung 12 abgezogen und zum einen als Rückfluss über die Leitung 13 in die Abtriebskolonne 2 geleitet und zum anderen als Produkt-Benzol 14 aus der Reinigungsvorrichtung abgezogen. Im Rückflussbehälter 10 löst sich der Rest der Verunreinigungen in der wässrigen Lösung. Aus der Abscheidevorrichtung 11 wird die wässrige Lösung 15 über eine Trennschichtregelung 16 ausgeschleust und teilweise über die Leitung 17 zurück zur Eindüsung vor den Kondensator gepumpt. Über die Leitung 18 wird ein anderer Teil der wässrigen Lösung 15 als Abwasser zur Aufarbeitung in eine biologische Kläranlage abgeführt. Das Verhältnis dieser beiden Ströme in den Leitungen 17 und 18 ergibt sich aus dem Gehalt der in der wässrigen Lösung gelösten Schadstoffe und der Ansprüche an die Reinheit des Benzols im Einzelfall. Zu beachten sind hierbei die Löslichkeitsgleichgewichte der Verunreinigungen sowohl für die Phase der wässrigen Lösung als auch die des Benzols. Der ausführende Fachmann wird hierzu im Einzelfall Laborversuche anstellen. Hierbei kann sich im Einzelfall auch ergeben, dass nur reines Frischwasser über die Leitung 7 eingedüst werden darf und dass die im Rückflussbehälter 10 abgetrennte wässrige Lösung 15 vollständig entsorgt werden muss.

Der zur Eindüsung bestimmten wässrigen Lösung in der Leitung 17 wird zur Einstellung eines pH-Wertes im Bereich von 7 bis 7,5 Ameisensäure 19 zugegeben, wobei die Dosierung über eine pH-Wert-Regelungseinrichtung 20 erfolgt. Durch die pH-Wert-Erniedrigung fällt ein Lösungsmittelsalz 22 aus, welches im Filter 21 aus der wässrigen Lösung ausgeschieden wird. Hierdurch wird eine Anreicherung von bereits abgeschiedenen Verunreinigungen in der wässrigen Lösung verhindert. Über die Leitung 23 wird die wässrige Lösung ergänzt um Wasser aus der Frischwasserleitung 24, um den Kreislauf um dasjenige Wasser aufzufüllen, welches gelöst im Produkt-Benzol 14 oder als Abwasser über die Leitung 18 die Reinigungseinrichtung verlässt. Um den Kondensationseffekt in der Mischzone 5 zu verstärken, kann die wässrige Lösung bei Bedarf im Wasserkühler 25 gekühlt werden.

### Bezugszeichenlegende:

- 1: Leitung für Benzol-Lösungsmittelgemisch
- 2: Abtriebskolonne
- 3: Leitung für dampfförmiges Benzol
- 4: Leitung für Lösungsmittel
- 5: Mischzone
- 6: Kondensator
- 7: wässrige Lösung
- 8: Leitung
- 9: Leitung für Kondensat
- 10: Rückflussbehälter
- 11: Wasserabscheidevorrichtung
- 12: Leitung für gereinigtes Benzol
- 13: Leitung für Rückfluss
- 14: Produkt-Benzol
- 15: wässrige Lösung
- 16: Trennschichtregelung
- 17: Leitung für Kreislauflösung
- 18: Leitung für Abwasser
- 19: Ameisensäure
- 20: pH-Wert-Regelungseinrichtung
- 21: Filter
- 22: Lösungsmittelsalz
- 23: Leitung
- 24: Frischwasserleitung
- 25: Wasserkühler

## Patentansprüche

1. Verfahren zur Reinigung eines Kohlenwasserstoffdampfes, der wenigstens aus einer aromatischen oder einer olefinischen oder einer paraffinischen Verbindung oder aus einer daraus gebildeten Mischung besteht und welcher Kohlenwasserstoffdampf verunreinigende Bestandteile mit sich führt, die sauren und/oder basischen Charakter besitzen und welche Bestandteile wenigstens aus einem wasserlöslichen, organischen und/oder anorganischen Stoff bestehen, **dadurch gekennzeichnet, dass** in den Kohlenwasserstoffdampf in einer Mischzone eine wässrige Lösung dispers eingebracht wird, der dadurch gebildete zweiphasige, mit Tröpfchennebel beladene Dampf unmittelbar in eine Kondensationszone übergeleitet wird, worin er mit beiden Phasen gemeinsam niedergeschlagen wird und die entstandene Kondensat-Emulsion in eine Abscheidezone eingeleitet wird, in der die im Wesentlichen gereinigten Kohlenwasserstoff enthaltende eine Teilflüssigphase der Emulsion von der im Wesentlichen Lösungswasser mit den darin gelösten verunreinigenden Bestandteilen enthaltenden anderen Teilflüssigphase der Emulsion abgetrennt wird.

2. Verfahren nach Anspruch 1, **dadurch gekennzeichnet, dass** die Abtrennung der einen, im Wesentlichen gereinigten Kohlenwasserstoff enthaltenden Teilflüssigphase der Emulsion von der anderen, im Wesentlichen Lösungswasser enthaltenden Teilflüssigphase der Emulsion mittels eines Wasserabscheiders erfolgt.

3. Verfahren nach einem der vorangegangenen Ansprüche 1 bis 2, **dadurch gekennzeichnet, dass** zumindest ein Teil des aus der Emulsion abgetrennten Lösungswassers in einem Kreislauf rückgeführt wird und in die besagte Mischzone als Bestandteil der wässrigen Lösung dispers eingebracht wird und mit dem verbliebenen Rest des aus der Emulsion abgetrennten Lösungswassers die besagten verunreinigenden Bestandteile aus dem Lösungswasserkreislauf ausgeschleust und ihrer Entsorgung zugeführt werden.

4. Verfahren nach einem der vorangegangenen Ansprüche 1 bis 3, **dadurch gekennzeichnet, dass** zumindest ein Teil der in die Mischzone dispers eingebrachten wässrigen Lösung aus Frischwasser besteht.

5. Verfahren nach einem der vorangegangenen Ansprüche 1 bis 4, **dadurch gekennzeichnet, dass** der in die Mischzone dispers eingebrachten wässrigen Lösung eine Säure beigemischt wird.

6. Verfahren nach Anspruch 5, **dadurch gekennzeichnet, dass** die der wässrigen Lösung beigemischte Säure Ameisensäure ist.

7. Verfahren nach Anspruch 5 oder 6, **dadurch gekennzeichnet, dass** die nach der Säurezugabe in der wässrigen Lösung ausgefällten Salze durch Abscheidemittel aus der wässrigen Lösung entfernt werden.

8. Verfahren nach den vorhergehenden Ansprüchen 5 bis 7, **dadurch gekennzeichnet, dass** die Säurezugabe pH-Wert-geregelt erfolgt.

9. Verfahren nach einem der vorangegangenen Ansprüche 1 bis 8, **dadurch gekennzeichnet, dass** die wässrige Lösung vor ihrem dispersen Einbringen in die Mischzone gekühlt wird.

10. Verfahren nach einem der vorangegangenen Ansprüche 1 bis 9, **dadurch gekennzeichnet, dass** die in der Kondensationszone entstandene Kondensat-Emulsion vor ihrer Einleitung in die Abscheidezone unterkühlt wird.

11. Vorrichtung zur Durchführung des Verfahrens nach mindestens einem der vorangegangenen Ansprüchen 1 bis 10, **dadurch gekennzeichnet, dass** die Mischzone und die Kondensationszone in einem beide Zonen einhüllenden gemeinsamen Raum angeordnet sind und dieser Raum von dem Mantel einer Einzelapparatur integrierend umgrenzt wird.
